# EUROPEAN PATENT APPLICATION

(11) **EP 1 153 573 A2**
(43) Date of publication of application: **14.11.2001**
(21) Application number: 01304203.1
(22) Date of filing: 10.05.2001
(51) Int. Cl.: A61B 7/04

(54) **Stethoscope mouse**

(30) Priority: 13.05.2000 KR 2000025689
(71) Applicant: Kim, Jaenam, Namdong-gu, Incheon 405-245 (KR); Ledart Co., Ltd., Gangnam-gu, Seoul 135-280 (KR)
(72) Inventor: Kim, Jaenam, Namdong-gu, Icheon 405-245 (KR)
(74) Representative: Neill, Alastair William

(57) **Abstract**

A stethoscope mouse (10) including a mouse and a stethoscope comprises a case (13); a mouse for inputting the position-detecting signals of the case and generating the control signal of a computer (191) related thereto and a system; a stethoscope integrated with the mouse in the case for collecting physiological information to represent it in a visual and/or aural manner on accessories of the system, transmit it to the computer for checking up it though a monitor and a speaker and diagnose the counterpart to be benefited from a physician at a remote place, if the computer transmits the physiological information data to another computer using a public communication network; and a selecting switch for generating the control signal of the system to integrate the inputting means and the diagnosing means and separate them from each other.

## Description

### BACKGROUND OF THE INVENTION

### Field of the Invention

The invention generally relates to a stethoscope, and particularly, to a computer mouse having a stethoscope for enabling the remote diagnosis and health care or consultation. More particularly, the invention is related to providing an stethoscope mouse connected to an on-line computer for performing the detection of sounds, for example the heart sound arising within the body and its phonocardiograph drafting, a body temperature measuring and its drafting and a skin conductivity measuring and its drafting and then enabling the computer to process them, obtaining physiological information, drafting them, transferring their data to a physician via the on-line communication.

### Description of the Background

In general, a mouse assembly is an inputting device of a computer, which is requisite to a personal system. All of computer application program supports the use of the mouse but also the local area network, public network or Internet communication of the computer is also subject to the utilization of the mouse.

A stethoscope is a kind of a diagnosis device, which enables a doctor to directly listen to sounds arising with the body and to check up the normal or abnormal condition. The stethoscope converts the physiological information such as a phonocardiographic sound, a body temperature, a skin conductivity etc., into an electric signal, so that the physician can obtain the physiological data through it, thereby providing the exact diagnosis results. A physician to provide his professional service to a patient in a face-to-face base uses such electronic stethoscopes, but it is not possible to use the stethoscope at the position where the physician is far away from the person being examined. A related typical technique is disclosed in U.S. Patent No. 5,025,809 which is related to a recording, digital stethoscope for identifying PCG signatures together with a computer and a display device for storing, analyzing and displaying the detected sounds together with comparative ones selected from a reference library.

Furthermore, keeping up with the wide spread of the personal computer and the expansion of the communication service, the on-line medical service is being offered for a remote diagnosis, examination, health care and consultation. In other words, a professional medical provider is designed to diagnose a patient at a remote place dependent upon information obtained from voice interaction via a telephone and a computer communication and image information via an Internet phone and a videophone. But, it means that the acquisition of the exact physiological information from the patient is difficult. Lacking of the information would leads to the distortion of the substantial condition. An example of this type of electronic stethoscope is U.S. Patent No. 6,014,432 which discloses a home health care system comprising a patient station and a health care provider's station, wherein the patient station includes a first video phone, an electronic imaging assembly and a stethoscope assembly coupled to the first video phone for respectively producing digital image and physiological sound signals of a patient, the first video phone simultaneously transmits the digital signals over a public telecommunication network. The stethoscope assembly comprises a general stethoscope and a processor including a stethoscope processing circuit for converting patient physiological sounds picked up by the stethoscope into compressed digital data transmitted by videophone over a network to the health care provider's station. The health care provider's station includes a second video phone, a video display and a sound reproducer for respectively receiving digital signals, displaying images of the patient on the display and reproducing the physiological sounds of the patient. Particularly, the home care provider's station requires additional inputting devices such as a stethoscope, a blood pressure device, a thermometer, a weight scale, a sphygmometer, etc., so the home care provider's station requires considerable skill to use.

In light of these problems, it is very preferable that the inputting device be simplified and becomes convenient to use. Also, it is very innovative if the real-time transmit occurs while being diagnosed.

Therefore, a main object of the invention is to provide an stethoscope mouse simply connected to public communications for providing physiological information of a person to be diagnosed to a medical service center.

The other object of the invention is to provide a stethoscope mouse including a physiological information inputting device and a stethoscope for respectively controlling the transmission of the physiological information on the on-line communication and collecting, processing and displaying the physiological data from a person to be benefited from the diagnosis of a physician under its system control.

Another object of the invention is to provide a stethoscope mouse connected to an on-line computer for listening to heart sounds and displaying its result, measuring a body temperature and displaying its result and measuring a skin conductivity, displaying its result and transmitting these physiological information to a physician via the on-line communication.

### SUMMARY OF THE INVENTION

According to the invention, a stethoscope mouse comprises a case; means for inputting the position-detecting signals of the case and generating the control signal of a computer related thereto and a system; means integrated with the inputting means in the case for collecting physiological information to represent it in a visual and/or aural manner through accessories of the system, transmit it to the computer and diagnose a counterpart to be benefited from a physician at a remote place, if the computer transmits the physiological information data to another computer using a public communication network; and means for generating the control signal of the system to integrate the inputting means and the diagnosing means and separate them from each other.

The inputting means including a mouse for detecting the X and Y coordinate movements of a mouse ball and a mouse controller means for receiving the position detecting signal of the mouse ball and the click signals of at least two mouse buttons and converting analog signals received into digital signals; the diagnosing means including a stethoscope means contacted with a human body for measuring heart sounds, body temperatures and skin conductivities, a microprocessor electrically connected to the stethoscope means for converting analog signals inputted thereinto into digital signals and processing converted data to control its storing, displaying and outputting operations in the system, a serial communication means connected thereto for transmitting the data inputted from the mouse controller means and the physiological information data digitally-converted to a computer and an earphone means including at least one earpiece for enabling the counterpart to listen to the heart sounds measured; and the control signal generating means connected to the microprocessor for determining the control mode of the system.

The stethoscope means includes an auscultation head and a microphone mounted therein, a body temperature sensor mounted on the outside of the auscultation head and three electrodes fixed mounted on the outside of the auscultation head for measuring the skin conductivities.

The control signal generating means includes a selecting switch for enabling the inputting signals of the mouse from the mouse controller and signals of heart sounds, body temperatures and skin conductivities to be selectively processed.
The microprocessor further comprises an aural frequency-converting portion for enabling the listening of heart sounds and a display portion for representing the physiological information measured. Also, the microprocessor transmits the data digitally-converted through the serial communication means to a computer in order to provide the physiological information to a user in a visual and aural manner by a monitor and a speaker. The microprocessor controls the computer to transmit the physiological data measured to another computer via a computer communication network, thereby enabling the diagnosis of a patient at a remote place in real time.

### BRIEF DESCRIPTION OF THE DRAWINGS

The invention now will be described in detail in reference to the accompanying drawings, in which:
Fig. 1 is a block diagram illustrating the principal components of a stethoscope mouse according to the invention;
Fig. 2 is a view illustrating the coupling state of an stethoscope mouse according to the invention;
Fig. 3A is a plan view illustrating the inner part of an stethoscope mouse according to the invention;
Fig. 3B is a side view illustrating the inner part of an stethoscope mouse according to the invention;
Fig. 4A is a bottom view illustrating an auscultation head coupled to an stethoscope mouse according to the invention; and
Fig. 4B is a side view illustrating an auscultation head coupled to an stethoscope mouse according to the invention.

### DETAILED DESCRIPTION OF THE PREFERRED EMBODIMENT

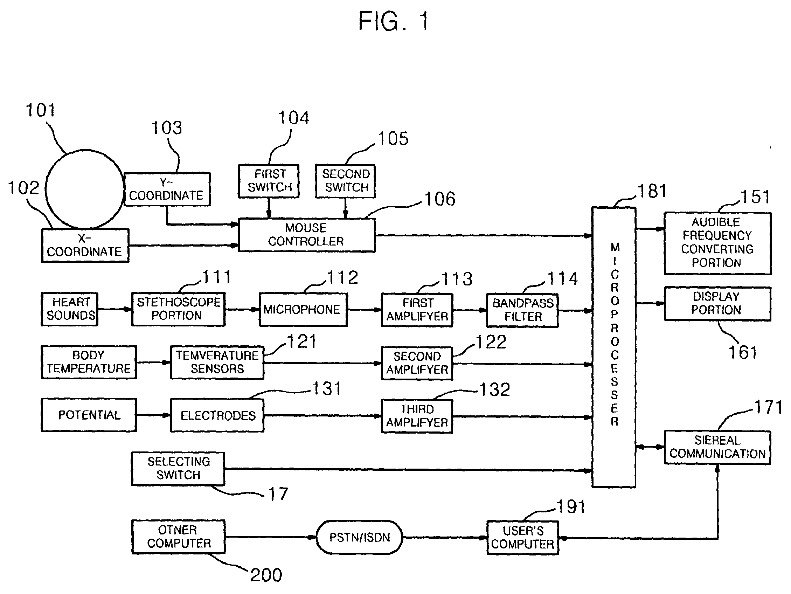

Referring to Fig. 1, the schematic block diagram of a stethoscope mouse 10 is illustrated. The stethoscope mouse 10 is subject to performing the inherent function of a mouse. As a mouse ball 101 is moved, the position information of the mouse ball 101 is detected by a sensor 102 of X -coordinate and a sensor 103 of Y-coordinate to apply the signals detected to a mouse controller 106. Switches 104 and 105 separately operated with the pressing and returning of a first button 11 and a second button 12 are turned on or off, the operation information of which are inputted to the mouse controller 106.

The mouse controller 106 converts the inputted signal into digital signals to be inputted to a microprocessor 181. The microprocessor 181 processes the digital signals and applies a control signal through a serial communication portion 171 to a personal computer 191. Therefore, the stethoscope mouse 10 enables the movement of a mouse pointer, the scroll movement of a screen, the click or double-click operation of an icon, etc. on the monitor of the personal computer 191. It means that the stethoscope mouse 10 controls the personal computer 191 to be connected through a public communication such as a PSTN and an ISDN to another computer 200, for example a target computer owned by a physician.

Also, the stethoscope mouse 10 comprises at least three means for obtaining physiological information of sounds arising within a body, body temperatures and skin conductivities. A stethoscope portion 111 includes an auscultation head 21 as described below in detail for picking up sounds from the organizations of a human body, a microphone 112 positioned in the stethoscope portion 111 to convert the picked-up sounds into the electric energy, a first amplifier 113 for amplifying signals outputted from the microphone 112 and a band pass filter 114 for removing noises. A temperature measuring portion includes a temperature sensor 121 properly positioned on the surrounding portion of the stethoscope cup 111 to measure the temperature of a human body and a second amplifier 122 for amplifying signals outputted from the temperature sensor 121. A skin conductivity measuring portion includes at least three electrodes 131 properly positioned on the surrounding portion of the stethoscope cup 111 to measure skin conductivities and a third amplifier 132 for amplifying electric potential signals of a human body outputted from the electrodes 131.

The microprocessor 181 receives the signals of the mouse controller 106 and the signals from the band pass filter 114, the first and second amplifiers 122 and 132 to be converted into digital signals and store their physiological information in its memory. The microprocessor 181 furthermore comprises an audio frequency converting portion 151 for converting digital signals into analog signals of an audible frequency, a display portion 161 for displaying the physiological data obtained by the stethoscope portion, the temperature sensors and the electrodes and a selecting switch 17 for enabling the selection of any one of the stethoscope and mouse functions.

Therefore, the stethoscope mouse 10 can use the stethoscope function and the mouse function for respectively transmitting data inputted into the microprocessor 181 to the personal computer 191 via the serial communication portion 171 and controlling the personal computer 191 to transmit the physiological information to the target computer 200 via PSTN or ISDN.

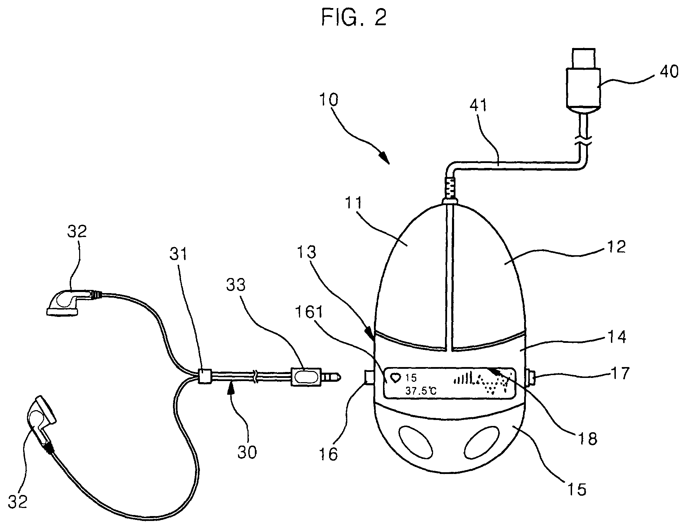

Referring to Fig. 2, the stethoscope mouse 10 is illustrated together with its accessories. The stethoscope mouse 10 comprises a case 13 having an arbitrary shape easy to grasp with a hand and convenient to perform the mouse function. The case 13 is shaped like a beetle species, wherein a first and second buttons 11 and 12 resemble beetle wings in the shape, an upper housing 14 resembles a beetle body in the shape, an auscultation head protection cap 15 resembles a beetle head in the shape, which is removably mounted on the case 13 to enable an auscultation head to be exposed for the use purpose of the stethoscope. The case 13 includes an upper housing 14 on the middle portion area of which a transparent window 18 having corners rounded in a circle shape is mounted to see a display portion 161.

The stethoscope mouse 10 is also provided with a plug 40 coupled with a mouse port of the personal computer 191 and a cable 41 connected to make a communication with the personal computer 191 which are wire communication components of the serial communication portion 171. Therefore, the stethoscope mouse 10 transmits physiological information signals through the plug 40 and the cable 41 to the personal computer 191. The personal computer 191 operates its monitor and speaker to give the physiological information to a user and transmit it to the target computer 200 via PSTN or ISDN as well. The serial communication portion 171 would be connected to a wireless communication device using infrared rays or a radio wave.

The selecting switch 17 is provided on the housing 14 projected from one side surface thereof to select the stethoscope function. An earphone connecting port 16 electrically connected to the audible frequency-converting portion 151 is mounted on the other side surface of the housing 14.

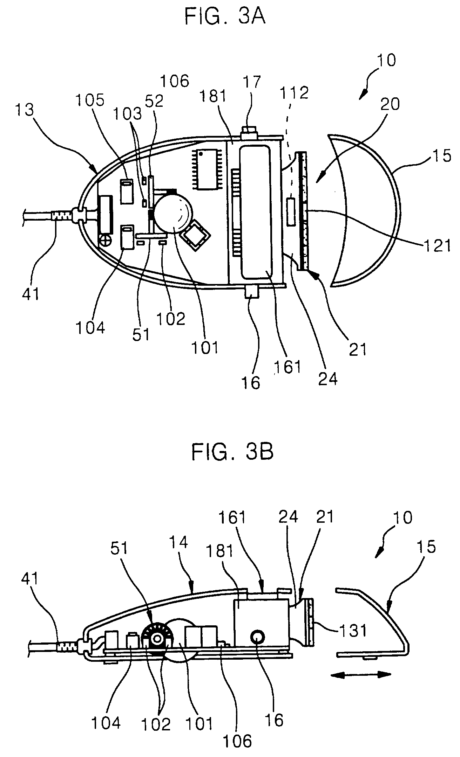

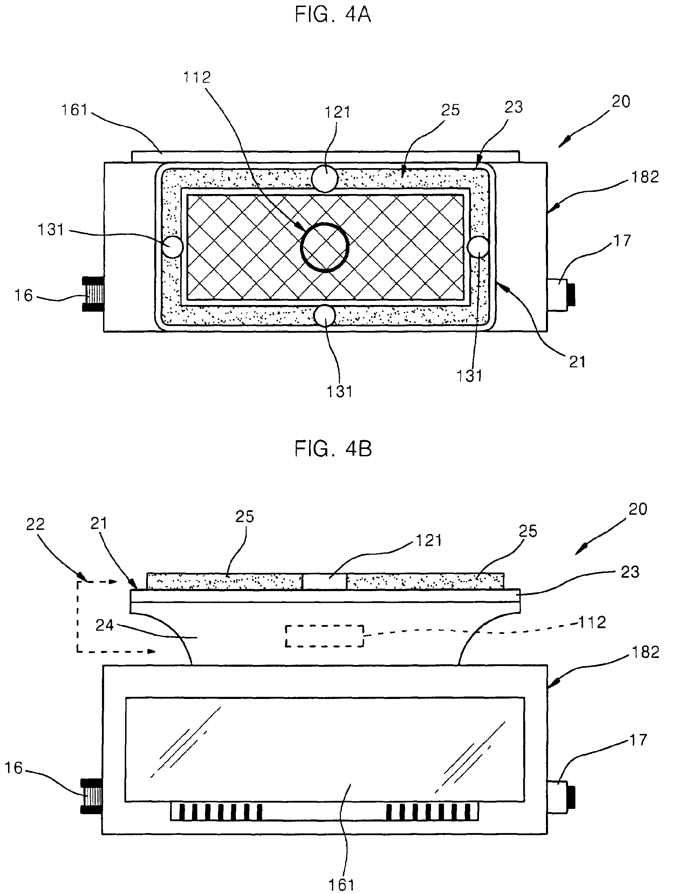

An earphone 30 includes earpieces 32 and 33 divided at a branch member 31 to be connected through a cable to an earphone plug 33. The earphone plug 33 is electrically connected to the earphone connecting port 16 with being inserted thereto. The earphone pieces 32 convert the received electrical signals into mechanical vibrations to listen to the heart sounds. And, the case 13 comprises more components for performing the stethoscope and mouse functions, which will be referring to Figs 3A, 3B, 4A and 4B for the purpose of more detailed explanation.

The stethoscope mouse 10 comprises a general ball mouse assembly to provide a position inputting function to a computer. The position-inputting device includes a ball 101 freely rotated. A X-coordinate slit disk 52 and a Y-coordinate slit disk are rotatably contacted to the ball 101, and their rotating amounts are respectively detected by X and Y coordinate sensors 102 and 103 to be inputted to a mouse controller 106. The mouse controller 106 computes a movement amount of the stethoscope mouse 10 to control a mouse pointer or a cursor. As the first and second buttons 11 and 12 are pressed or released, the mouse controller 106 scans the signal from the first and second switches 104 and 105.

Therefore, the stethoscope mouse 10 can function as the general mouse assembly to move the pointer, scroll the screen and click or double-click an icon. The selecting switch 17 can select the mouse inputting function, while the electronic stethoscope the selecting switch 17, the components of which are mounted in the case 13, can select function. The auscultation head 21 is positioned on the head portion of the case 13, so that it is easily contacted to a body portion to be diagnosed when the auscultation head protection cap 15 is removed and the user properly grasps the case 13. At the same time, the earphone plug 33 is inserted into the earphone connecting port 16, so that the earpieces 32 enable the user to listen to his heart sounds. The auscultation head 21 has an approximate hone-shaped structure, which is a rectangular when seen from its front, and its opening surface is exposed to the outside.

Referring to Figs. 4A and 4B, the operating portions of the stethoscope mouse 10 are illustrated. A body-measuring device 22 comprises the auscultation head 21 including a hollow resonant chamber 24 of the horn-shaped structure and a microphone 112 positioned at the center of the chamber 24. A temperature sensor 121 and three electrodes 131 are properly mounted on a substrate 23, which is positioned on the front surrounding portion of the hollow resonant chamber. A body-contacting ring 25 is mounted around the circumference of the substrate 23. The hollow resonant chamber 24 is properly mounted at the connecting portion to a printed circuit board 182 of the microprocessor 181.

According to the invention, the auscultation head 21 is the rectangular shape which is slightly different from the circular shape of the general stethoscope assembly if seen from the body contacting surface, but may have the structure the same as that of the general stethoscope assembly. Therefore, the stethoscope portion 111 includes the substrate 23 mounting the body temperature sensor 121 and three electrodes 131, the hollow resonant chamber 24 for collecting the sounds of the body organizations and the microphone 112 positioned in the inner center of the hollow resonant chamber 24 for converting the resonant acoustic sounds into the electrical energies. The microphone 112 is mounted in the inner of the hollow resonant chamber 24 away from the contacting surface of the contacting ring 25 to be separated from the body portion to be diagnosed.

On the other hand, the temperature sensor 121 is positioned on an elastic member (not shown), which could be separately mounted on the substrate 23, so that it is subject to an appropriate pressure against the human body to secure the exact measurement of the body temperature. Also, the three electrodes 131 would be positioned on the elastic member for more certain body contacting and is preferably arranged in an Einthoven Triangle.

The body-measuring device 22 further comprises electronic components such as an amplifier, a band pass filter and an analog/digital converter for receiving electrical signals therefrom. The analog signals from the first and second amplifier 113 and 122 and the band pass filter 114 are amplified and/or filtered in turns, converted into digital signals by means of the analog/digital converters (not shown) and then inputted to the microprocessor 181. Herein, it is noted that according to the invention the microprocessor 181 includes analog/digital converters mounted therein. Therefore, the microprocessor 181 acts to store digital data converted by its analog/digital converter in its memory and control the system on the basis of the digital data. The control function contains the selective control of the mouse and stethoscope functions by the operation of the selecting switch 17.

Also, the microprocessor 181 converts the measured data of heart sounds, body temperatures, skin conductivities, etc. into screen information of characters or graphics and inputs them to the display portion 161 to be represented immediately. Furthermore, the first and second buttons 11 and 12 having an inputting function can be operated to select the wave form of the heart sounds or the skin conductivities so as to represent their visual information on the display portion 161 or to provide a display mode changing function such as a sample & hold function for instantly capturing varying data and representing it on the display portion 161, etc. Therefore, the display mode changing function using the first and second buttons 11 and 12 can be performed in various manners by the selecting switch 17.

The microprocessor 181 comprises the audible frequency converting portion 151 for enabling the user to listen to the digital data of the heart sounds, pretending that the microprocessor 181 receives the digital data of the heart sounds, reconverts it into the analog signals and then outputs the electrical vibration signals to the earphone connecting port 16. The volume of the electrical vibration signal is adjusted by the operation of the first and second buttons 11 and 12 that can be constituted as a volume adjuster. Also, the band pass filter 114 can be selected to have a predetermined bandwidth to listen to the heart sound of a specific frequency. The insertion of the earphone plug 33 into the earphone connecting port 16 establishes the electrical circuit of the earphone 30, so that the electrical vibration signals from the audible frequency converting portion 151 are applied to the earpieces 32 to be converted into the mechanical vibration through its speaker, thereby enabling the user to listen to the heart sounds.

The earphone 30 includes two earpieces 32 using the Y-shaped branch member 31, but it has the same effect through only one earpiece 32 is provided with the branch member 31 not being adopted. The earphone 30 can be separated from the earphone connecting port 16 to enhance the convenience only for the mouse function, when the stethoscope mouse 10 is used as a mouse-inputting device.

The microprocessor 181 comprises the serial communication portion 171 for transmitting the data of the mouse and auscultation functions and their commands integrated in it to the personal computer 191. The personal computer 191 processes the data from the serial communication portion 171 to display the information of heart sounds or the body temperatures and the skin conductivities on its monitor, or generate the heart sounds by a speaker, thereby enabling the stethoscope mouse 10 to provide the physiological information in the visual and aural manner to the user. Also, the personal computer 191 transmits the measured data via the communication network such as PSTN, ISDN, etc. to another computer 200 based on its communication programming in order to co-own the fundamental physiological information with each other, thereby enabling the physician to gain the diagnosis materials at a remote place.

### Effect of the Invention

As it is clear from the above explanation, according to the invention a stethoscope mouse integrates the inputting function of a computer mouse and the diagnosis function of a stethoscope to provide the operation of the computer and the electronic stethoscope function without mounting additional stethoscope or mouse.

The stethoscope mouse simply processes the physiological-electrical vibration signals measured by the measuring components to generate the visual and audible outputting signals and evaluate and sample-hold them. At the same time, the stethoscope mouse enables the computer communication using the mouse inputting function to enhance the fundamental diagnosis quality at the remote place through the information co-owned in real time.

Particularly, the physician can gain data measured by the stethoscope mouse use of a patient or his protector at the remote place according to his instruction in real time, whereby the critical abnormal information related to the physiology is checked up at the first stage to enhance the quality of the remote diagnosis service. Also, the measured data can be transmitted/stored to and in a personal computer, immediately, and then analyzed.

The stethoscope mouse enables the multilateral communication to observe the physiological information by a plurality of physicians at the same time, therefore reducing the possibility of erroneous diagnosis caused due to the stethoscope error.

The reader's attention is directed to all papers and documents which are filed concurrently with or previous to this specification in connection with this application and which are open to public inspection with this specification, and the contents of all such papers and documents are incorporated herein by reference.

All of the features disclosed in this specification (including any accompanying claims, abstract and drawings), and/or all of the steps of any method or process so disclosed, may be combined in any combination, except combinations where at least some of such features and/or steps are mutually exclusive.

Each feature disclosed in this specification (including any accompanying claims, abstract and drawings), may be replaced by alternative features serving the same, equivalent or similar purpose, unless expressly stated otherwise. Thus, unless expressly stated otherwise, each feature disclosed is one example only of a generic series of equivalent or similar features.

The invention is not restricted to the details of the foregoing embodiment(s). The invention extend to any novel one, or any novel combination, of the features disclosed in this specification (including any accompanying claims, abstract and drawings), or to any novel one, or any novel combination, of the steps of any method or process so disclosed.

## Claims

1. A stethoscope mouse including a mouse and a stethoscope comprising;
a case;
means for inputting the position-detecting signals of the case and generating the control of a computer related thereto and a system;
means integrated with the inputting means in the case for collecting physiological information to represent it in a visual and/or aural manner through accessories of the system, transmit it to the computer for checking up it on a monitor and a speaker and diagnose a counterpart to be benefited from a physician at a remote place, if the computer transmits the physiological information data to another computer using a public communication network; and
means for generating the control signal of the system to integrate the inputting means and the diagnosing means and to separate them from each other.

2. The stethoscope mouse according to Claim 1, wherein;
the inputting means including a mouse for detecting the X and Y coordinate movements of a mouse ball and a mouse controller means for receiving the position-detecting signal of the mouse ball and the click signals of at least two mouse buttons and converting received analog signals into digital signals;
the diagnosing means including a stethoscope means contacted with a human body for measuring heart sounds, body temperatures and skin conductivities, a microprocessor electrically connected to the stethoscope for converting analog signals inputted thereinto into digital signals and processing the converted data to control its storing, displaying and outputting in the system, a serial communication means connected to the microprocessor for transmitting the data inputted from the mouse controller means and the physiological information data digitally-converted and an earphone means including at least one earpiece for enabling the counterpart to listen to the heart sounds measured; and
the control-generating means connected to the microprocessor for determining the control mode of the system.

3. The stethoscope mouse according to Claim 2, wherein;
the stethoscope means includes an auscultation head and a microphone mounted therein, a body temperature sensor mounted on the outside of the auscultation head and three electrodes fixed on the outside of the auscultation head for measuring the skin conductivities.

4. The stethoscope mouse according to Claim 3, wherein;
the stethoscope mouse further comprises an auscultation head protection cap.

5. The stethoscope mouse according to Claim 2, wherein;
the microprocessor further comprises an audible-frequency-converting portion for converting the digital data measured into analog signals to listen to heart sounds and a display portion and an earphone for representing the physiological information measured in visual and aural manners, respectively and the control signal generating means including a selecting means for transmitting the data represented on the display portion by operating the mouse buttons to another computer via the communication network.

6. The stethoscope mouse according to Claim 2, wherein;
the serial communication means includes a wire cable and a communicating-port-connecting plug for communicating with the computer.
